# EUROPÄISCHE PATENTANMELDUNG

(11) **EP 1 522 252 A1**
(43) Veröffentlichungstag der Anmeldung: **13.04.2005**
(21) Anmeldenummer: 04022459.4
(22) Anmeldetag: 21.09.2004
(51) Int. Cl.: A61B 1/05, A61B 18/24, A61B 19/00

(54) **Verfahren zur automatisierten Lokalisierung von Läsionen im Gastrointestinaltrakt zur Therapie mit Laserlicht**

(30) Priorität: 06.10.2003 DE 10346276
(71) Anmelder: SIEMENS AKTIENGESELLSCHAFT, 80333 München (DE)
(72) Erfinder: Abraham-Fuchs, Klaus, 91058 Erlangen (DE); Fuchs, Friedrich, Dr., 91341 Röttenbach (DE); Kuth, Rainer, 91074 Herzogenaurach (DE)

(57) **Zusammenfassung**

Die vorliegende Erfindung bezieht sich auf ein Verfahren zur automatisierten Lokalisierung von Läsionen (1) im Gastrointestinaltrakt mit Laserlicht eines Endoroboters (5), aufweisend die folgenden Schritte:
- Aufnehmen von Oberflächenbildern des Gastrointestinaltraktes mit der Kamera (7) des Endoroboters (5)
- Übermitteln der Oberflächenbilder an einen Rechner (13)
- Analysieren der Oberflächenbilder durch den Rechner (13) nach zu bestrahlenden Läsionen auf Basis läsionspezifischer Merkmale
- Berechnen von Regelsignalen durch den Rechner (13) auf Basis einer erkannten Läsion (1),
- Übermitteln der Regelsignale an ein Endoroboter-Steuergerät und/oder an ein im Endoroboter befindliches Lasersystem (10), und
- Einstellen der berechneten Laserstrahl-Ausrichtung und Laserbestrahlungsflächengröße durch das Endoroboter-Steuergerät und/oder durch das Lasersystem (10) auf Basis der Regelsignale.

## Beschreibung

Die vorliegende Erfindung bezieht sich allgemein auf die Endoskopie mit einem Endoroboter zur Durchführung minimal invasiver Diagnosen und Eingriffe im Körperinneren, vorzugsweise im Gastrointestinaltrakt eines Patienten. Dabei bezieht sich die vorliegende Erfindung insbesondere auf ein Verfahren sowie ein System zur Durchführung des Verfahrens, um Läsionen in kurzer Zeit bedienerunabhängig zu identifizieren und mit Laserlicht zu bestrahlen.

Der Gastrointestinaltrakt (Magen-Darm-Trakt) des Menschen ist Schauplatz akuter und/oder chronischer Erkrankungen beispielsweise in Form pathologischer veränderter Stellen der Innenseite von Magen oder Darm, die sporadisch oder kontinuierlich bluten, zum Beispiel im Rahmen entzündlicher oder neoplastischer Prozesse.

Von besonderer Bedeutung sind entzündliche Darmerkrankungen wie Morbus Crohn, Colitis ulcerosa, neuroendokrine Tumore des Dünndarms, Tumore allgemein oder aber auch kleine Gewebeveränderungen, wie zum Beipiel Polypen, welche im Laufe der Zeit in hohem Maße maligne (bösartig) entarten. Oft sind diese im folgenden als (oberflächliche) Läsionen bezeichneten pathologischen Gewebeveränderungen beim selben Patienten an vielen Orten zu finden, die sich in der Regel zu bösartigen Darmkrebsen entwickeln, sofern deren Wachstum nicht rechtzeitig durch Entfernung oder anderweitige Zerstörung, beispielsweise durch Elektrokoagulation oder Koagulation mittels Laserlicht, vernichtet werden.

Eine Zerstörung von eben beschriebenen Läsionen durch Koagulation wird nach dem Stand der Technik mittels Endoskop bzw. mittels Endoroboters durchgeführt. Ein Endoroboter, der einen ablationsfähigen Laser aufweist, ist in der Patentschrift US 6,240,312 B1 beschrieben. Die Steuerung eines solchen Endoroboters im Körperinneren wird mittels Endoroboter-Steuergerät durch ein Magnetfeldsteuerungssystem realisiert und ist in der Patentschrift DE 101 42 253 C1 ausführlich dargestellt.

Aufgrund der enormen Länge des Darms (bis zu 11 Meter) und der üblicherweise großen Anzahl von Läsionen ist es Aufgabe der vorliegenden Erfindung, ein Verfahren bzw. ein System bereitzustellen, durch welches Läsionen im Gastrointestinaltrakt automatisch erkannt und automatisiert zerstört bzw. therapiert werden können.

Diese Aufgabe wird gemäß der Erfindung durch die Merkmale der unabhängigen Ansprüche gelöst. Die abhängigen Ansprüche bilden den zentralen Gedanken der Erfindung in besonders vorteilhafter Weise weiter.

Erfindungsgemäß wird daher ein Verfahren zur automatisierten Lokalisierung von Läsionen im Gastrointestinaltrakt mit Laserlicht eines Endoroboters beansprucht, aufweisend die folgenden Schritte:
- Aufnehmen von Oberflächenbildern des Gastrointestinaltraktes mit der Kamera des Endoroboters
- Übermitteln der Oberflächenbilder an einen Rechner
- Analysieren der Oberflächenbilder durch den Rechner nach zu bestrahlenden Läsionen auf Basis läsionspezifischer Merkmale
- Berechnen von Regelsignalen durch den Rechner auf Basis einer erkannten Läsion,
- Übermitteln der Regelsignale an ein Endoroboter-Steuergerät und/oder an ein im Endoroboter befindliches Lasersystem, und
- Einstellen der berechneten Laserstrahl-Ausrichtung und Laserbestrahlungsflächengröße durch das Endoroboter-Steuergerät und/oder durch das Lasersystem auf Basis der Regelsignale.

Ferner wird erfindungsgemäß ein Verfahren beansprucht welches ein geregeltes Bestrahlen der erkannten Läsion ermöglicht, aufweisend die folgenden Schritte:
- Bestrahlen der vom Rechner auf Basis läsionspezifischer Merkmale erkannten Läsion mit dem Laser des Endoroboters
- Messen aktueller läsionspezifischer Merkmale der bestrahlten Fläche durch die Kamera nach Abschalten des Lasers
- Ermitteln der Größe der Laserbestrahlungsfläche durch den Rechner auf Basis der gemessenen aktuellen läsionspezifischen Merkmale
- Einstellen der ermittelten Laserbestrahlungsflächengröße durch Positionieren des Endoroboters orthogonal zur Läsionoberfläche
- Kontinuierliches oder pulsartiges Bestrahlen der Läsionoberfläche, Messen und Überwachen der Größe sowie der aktuellen läsionspezifischen Merkmale der Laserbestrahlungsfläche auf der Läsionoberfläche.

Dabei wird vorteilhaft in einer ersten möglichen Ausführungsform der Erfindung die Laserbestrahlungsflächengröße in Abhängigkeit der gemessenen aktuellen läsionspezifischen Merkmale der Laserbestrahlungsfläche auf der Läsionoberfläche durch Einstellen der Entfernung des Endoroboters zur Läsionoberfläche variiert.

In einer zweiten möglichen Ausführungsform der Erfindung wird die Laserbestrahlungsflächengröße in Abhängigkeit der gemessenen aktuellen läsionspezifischen Merkmale der Laserbestrahlungsfläche auf der Läsionoberfläche durch Variation des Lasersystems variiert.

Erfindungsgemäß betreffen die läsionspezifischen Merkmale die Farbe, die Form, sowie die Textur einer spezifischen Läsion.

Vorteilhaft werden die läsionspezifischen Merkmale mit Läsionsmerkmalen in einer dem Rechner zugänglichen Bibliothek bzw. in einer Bibliothek des Rechners verglichen.

Erfindungsgemäß kann die Bestrahlung mit Laserlicht zur Messung der Laserbestrahlungsflächengröße sowie zur Messung der läsionspezifischen Merkmale jederzeit bzw. in periodischen Intervallen unterbrochen bzw. gestoppt werden.

Vorteilhaft erfolgt das Aufnehmen von Oberflächenbildern, das Übermitteln an den Rechner sowie das Analysieren durch den Rechner kontinuierlich.

In einer weiteren Ausgestaltung der Erfindung wird durch transversales Verschieben des Laserstrahles in der XY-Ebene bzw. durch Schwenken des Laserstrahles relativ zur Läsion-Endoroboter-Verbindungslinie (Z-Achse) durch entsprechende Bewegung des Endoroboters in Kombination mit dem kontrollierten Positionieren in Z-Richtung des Endoroboters die gesamte Läsionoberfläche mit definierter Flächenleistungsdichte des Lasers bestrahlt.

Dabei wird erfindungsgemäß die Flächenleistungsdichte in Abhängigkeit von der Art der Läsion über die Zeit vom Rechner vorgegeben.

Vorteilhaft erfolgt das transversale Verschieben des Laserstrahles in der XY-Ebene bzw. das Schwenken des Laserstrahles relativ zur Z-Achse durch räumliche Ausrichtung des Lasers relativ zum Endoroboter.

Dabei erfolgt die räumliche Ausrichtung des Lasers relativ zum Endoroboter in einer möglichen Ausführungsform durch miniaturisierte elektromechanische Mechanismen, wie bewegliche Membrane, Formgedächtnislegierungen, elektrisch kontraktierbare Polymere, Piezoaktoren usw.

In einer weiteren Ausgestaltung der Erfindung wird die Laserstrahlbewegung durch Verschiebung einer Blende bewirkt.

In einer dritten möglichen Ausgestaltung der Erfindung wird die Laserstrahlbewegung durch Aktivieren ausgewählter Laserdioden eines im Endoroboters integrierten 3D-Laser-Dioden-Arrays bewirkt.

In einer vierten Ausgestaltung der vorliegenden Erfindung wird die Laserstrahlbewegung durch ein Magnetfeldsteuerungssystem bewirkt, in dem der im Endoroboter integrierte Laser an einen beweglichen Miniaturmagneten bzw. an magnetisierbares Material gekoppelt ist, welcher bzw. welches nicht zur Navigation des Endoroboters dient, und welcher bzw. welches durch Anlegen eines äußeren Magnetfeldes geschwenkt bzw. verschoben werden kann.

Eine Ausrichtung weiterer Komponenten des Endoroboters, zum Beispiel die Kamera, erfolgt in einer weiteren Ausgestaltung der Erfindung mittels der Steuerprinzipen gemäß der Ansprüche 12 bis 15.

Ferner wird erfindungsgemäß ein System zur Durchführung eines Verfahrens nach einem der Ansprüche 1 bis 16 beansprucht.

In möglichen Ausführungsformen des erfindungsgemäßen Systems kann der Rechner entweder außerhalb des Patienten oder aber im Endoroboter integriert sein oder aber eine Kombination aus integrierten und ausserhalb platzierten Rechner darstellen.

Weitere Vorteile, Merkmale und Eigenschaften der vorliegenden Erfindung werden nun anhand von Ausführungsbeispielen bezugnehmend auf die begleitenden Zeichnungen näher erläutert.
Fig. 1 zeigt ein System, mit dem der Laserstrahl eines Endoroboters rechnergesteuert manövriert werden kann,
Fig. 2 zeigt einen laseraufweisenden Endoroboter in einem kartesischen Koordinatensystem relativ zu einer zu koagulierenden Läsion einer erkrankten Organoberfläche,
Fig. 3a zeigt einen ersten Regelkreis bezüglich der Laserbestrahlungsfläche auf der zu koagulierenden Läsion, und
Fig. 3b zeigt einen zweiten Regelkreis bezüglich der zweidimensionalen Abtastung der zu koagulierenden Oberfläche.

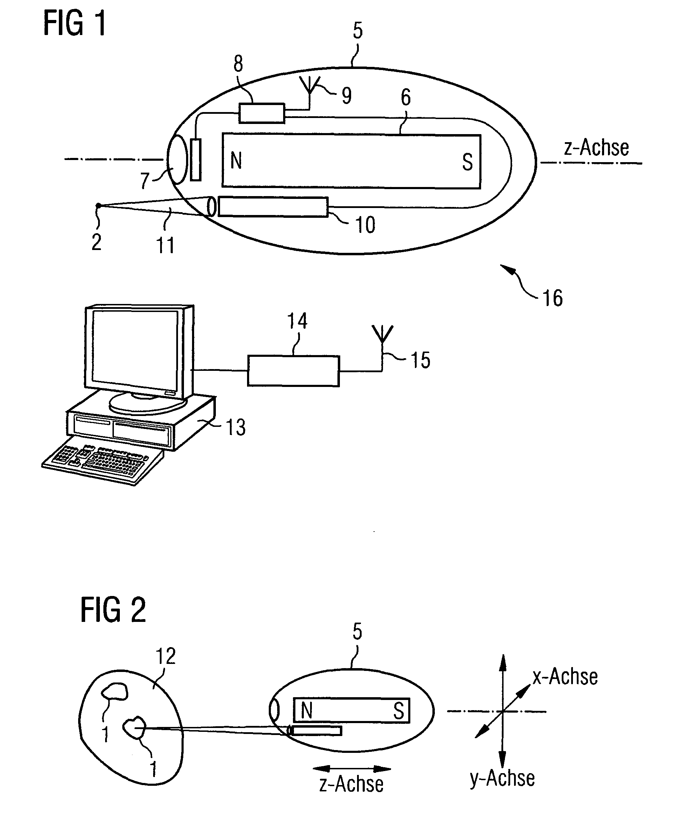

In Fig. 1 ist ein Endoroboter 5 dargestellt, der einen ablationsfähigen Laser 10 aufweist. Der austretende Laserstrahl 11 wird gebündelt und parallel zur Läsion-Endoroboter-Verbindungslinie (im folgenden als Z-Achse bezeichnet) fokussiert. Der Laserfokus 2 befindet sich üblicherweise in einigen Zentimetern Entfernung zum Endoroboter 5. Am laserstrahlseitigen Ende des Endoroboters 5 ist mittig eine (CCD-)Kamera mit Objektiv 7 angeordnet, durch die der fokusseitige Teil des Laserstrahles 11 optisch erfasst werden kann. Die Kamera 7 ist mit einer HF-Sende- und Empfangseinheit 8 verbunden, durch die über eine Antenne 9 des Endoroboters die aufgenommenen Bilder in Echtzeit über eine Antenne 15 der HF-Sendeund Empfangseinheit 14 eines Rechners 13 übermittelt wird. Die übermittelten Bilder können beispielsweise an einem Bildschirm des Rechners dargestellt und vom Anwender (z. B. dem Arzt) zur Diagnose oder zur Therapieplanung herangezogen werden.

Dabei erfolgt die Steuerung bzw. das Manövrieren des Endoroboters im Gastrointestinaltrakt über ein Magnetfeldsteuerungssystem (nicht dargestellt). Dazu ist der Endoroboter mit einem Linearmagneten 6 versehen, auf den in Wechselwirkung mit einem 3D-Gradientenfeld ein Drehmoment und eine Translationskraft so ausgeübt werden kann, dass der Endoroboter entlang beispielsweise des Darmes bewegt wird. Die Navigation erfolgt entweder durch den Anwender mit Hilfe eines Krafteingabegerätes (z. B. einer sogenannten 6D-Maus), durch das das 3D-Gradientenfeld entsprechend variiert werden kann. Eine andere Möglichkeit wäre eine rechnergesteuerte Navigation, in dem der Rechner über die Kamera des Endoroboters die Darmwand erkennt und das 3D-Gradientenfeld so steuert bzw. regelt, dass der Endoroboter Benutzer-unabhängig entlang des Darmes geführt wird.

Die vorliegende Erfindung besteht nun darin, das Endoroboter-Rechnersystem so auszugestalten, dass der Endoroboter über die Kamera Läsionen in der Darmwand erkennt und diese mit seinem Laser weitgehend Benutzer-unabhängig so bestrahlt, dass diese Läsionen letztendlich als therapiert angesehen werden können. Einer der wesentlichen Punkte der Erfindung ist die Automatisierung der Bestrahlung nach vorausgegangener, ebenso automatisch erfolgter Identifizierung der jeweiligen Läsion.

Das erfindungsgemäße System besteht zum einen aus einem kapselartigen Endoroboter 5 welcher einen Stabmagneten 6, eine Kamera 7 sowie einen ablationsfähigen Laser 10 aufweist. Der Stabmagnet 6 ist im Rahmen eines Endoroboter-Steuergerätes elektromagnetisch an dynamische Magnetfelder eines Magnetfeldsteuerungssystems gekoppelt wodurch der Endoroboter 5 z.B. mit einer 6D-Maus im Raum navigiert werden kann. Die dynamischen Magnetfelder werden durch Maxwell-Spulen erzeugt die um den zu untersuchenden Patienten angeordnet sind. Kamera 7 und Laser 10 des Endoroboters 5 sind an eine mit einer Antenne 9 verbundenen HF-Sende-und-Empfangseinheit 8 des Endoroboters 5 gekoppelt. Das System 16 besteht weiterhin aus einem Rechner mit Bildschirm 13. Der Rechner 13 ist ebenfalls mit einer HF-Sende-und-Empfangs-Einheit 14 und diese wiederum mit einer Antenne 15 verbunden, so dass insbesondere die von der Endoroboter-Kamera 7 (kontinuierlich) aufgenommenen Bilder an den Rechner 13 gesendet und in Echtzeit auf dessen Bildschirm dargestellt werden können. Auf Basis der Bilddarstellung auf dem Bildschirm des Rechners 13 wird der Endoroboter 5 vom Anwender entsprechend ausgerichtet bzw. positioniert und der Laser 10 entsprechend eingestellt.

In Fig. 2 ist die Vorgehensweise der Bestrahlung grob skizziert. Die Kamera 7 des Endoroboters erkennt eine Läsion 1 auf der erkrankten Organoberfläche 12, beispielsweise der Darmoberfläche. In einer vorteilhaften Ausgestaltung der Erfindung erfolgt das Erkennen einer Läsion 1 rechnerbasiert und wird später ausführlicher erläutert. Der Rechner 13 wirkt über das Magnetfeldsteuerungssystem auf den Endoroboter 5 so ein, dass durch ein Schwenken bzw. eine Translation in X- und Y-Richtung des Endoroboters 5 der Laserstrahl 11 auf die erkannte Läsion 1 gerichtet wird. Durch Verschieben des Endoroboters 5 entlang der Läsion-Endoroboter-Verbindungslinie (Z-Achse) kann die Größe der Laserbestrahlungsfläche und damit die Flächenleistungsdichte der Bestrahlung justiert bzw. eingestellt werden.

Die eben beschriebene initiale Ausrichtung des Endorotobers 5 durch den Rechner sowie die nachfolgende rechnergesteuerte Bestrahlung selbst erfolgt erfindungsgemäß automatisiert in Wechselwirkung mit der im Endoroboter integrierten Kamera 7. Die Kamera 7 liefert Bilder der erkannten Läsion 1, insbesondere aber der Laserbestrahlungsfläche, die über die HF-Sendeempfangseinheit 8 an den Rechner 13 in Echtzeit übermittelt werden. Die Bilder enthalten außer der Größe und Form der Laserbestrahlungsfläche Information über Farbe, Form und Textur der Läsion, sowie bereits bestrahlter Bereiche der Läsion. Auf Basis von Bilderkennungsalgorithmen ist der Rechner in der Lage, Farb-, Form- und Texturmerkmale vor und nach einer Bestrahlung spezifischen Läsionen (Läsionsklassen, Läsionstypen) zuzuordnen, die in einer vorteilhaften Ausgestaltung der vorliegenden Erfindung in einer dem Rechner 13 zugänglichen Bibliothek abgelegt sind.

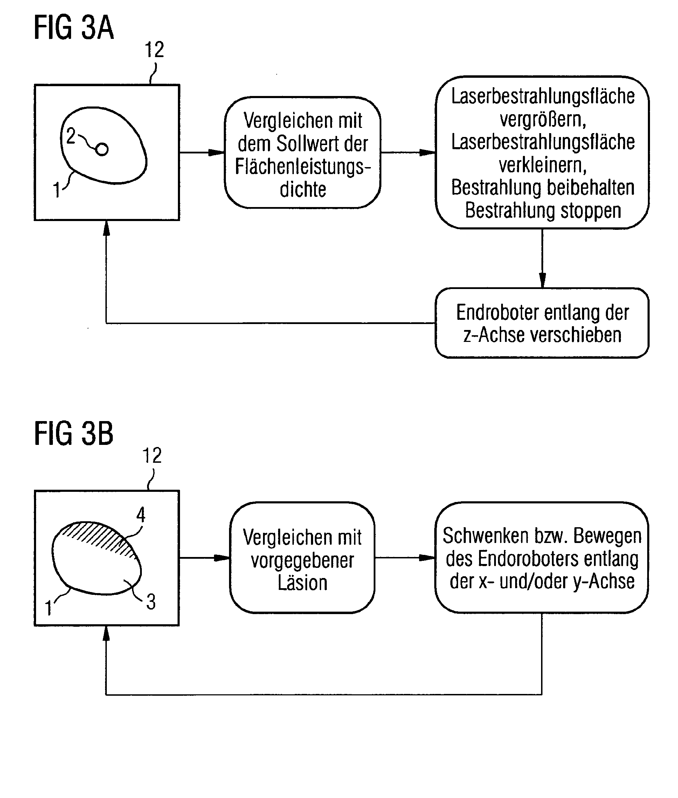

Die rechnerbasierte Spezifizierung bzw. Klassifizierung der Läsion (des Läsionstyps) nach ihrer Erkennung ermöglicht eine Optimierung des anschließenden Bestrahlungsverlaufes hinsichtlich Größe der Laserbestrahlungsfläche, Bestrahlungsdauer sowie Laserlichtfrequenz. Diesem Bestrahlungsverlauf sind im wesentlichen zwei Regelkreise zugrundegelegt:
Regelkreis 1 regelt die Größe der Laserbestrahlungsfläche bzw. die Flächenleistungsdichte über den Abstand des Endoroboters 5 zur Läsionoberfläche sowie über die gewählte Laserfrequenz. Üblicherweise ist der Laser 10 eines Endoroboters 5 mit einer Laserspeichergeräten (Speicherung auf optischen Platten) ähnlichen Optik ausgestattet, wodurch in einer endlichen Entfernung vom Endoroboter 5 bis zum Gewebe die optische Leistung auf eine minimale Fläche (ein Mikrometer) konzentriert werden kann. Damit ergibt sich die Möglichkeit, bei einer an sich starren Optik über die Entfernung zwischen Laser 10 bzw. Endoroboter 5 und der zu behandelnden bzw. zu bestrahlenden Läsion 1 die Flächenleistungsdichte definiert (gesteuert) zu verändern. Die Größe der Laserbestrahlungsfläche 2 wird über die Kamera 7 des Endoroboters 5 periodisch oder kontinuierlich beobachtet und einem Regelkreis im Rechner 13 zugeführt, welcher den Abstand eben so einstellt, dass die grundsätzlich von der Art der Läsion 1 abhängige optimale Flächenleistungsdichte erreicht wird.
Der Regelkreis 1 ist in Fig. 3a dargestellt. Die Art der Läsion 1 auf Basis von Form, Größe, Farbe, Textur wird vor, während oder nach einer Bestrahlung mit Laserlicht bestimmt, in dem durch den Rechner 13 die genannten Merkmale mit den entsprechenden, in einer Bibliothek vorliegenden Merkmalen unterschiedlicher Läsionstypen verglichen werden. Der Läsionstyp ist mit einem Sollwert der Flächenleistungsdichte verknüpft, die durch Vergrößern oder Verkleinern der Laserbestrahlungsfläche aber auch durch Beibehalten bzw. Stoppen der Bestrahlung eingestellt werden kann. Die Einstellung erfolgt erfindungsgemäß rechnerbasiert. Dabei ist die Laserbestrahlungsfläche 2 üblicherweise um ein vielfaches kleiner als die Läsion 1 selbst. Um dennoch die gesamte Läsion 1 eventuell mit Saum automatisiert bzw. rechnergesteuert zu bestrahlen, ist ein weiterer zweiter Regelkreis notwendig, der bewirkt, dass die gesamte Läsion 1 (eventuell mit Saum) von dem Laserstrahl abgetastet wird.
Ein solcher zweiter Regelkreis ist in Fig. 3b dargestellt. Die Kamera 7 des Endoroboters 5 erfasst kontinuierlich oder periodisch die Läsionsfläche 1 des erkrankten Organes 12. Die Sende- und Empfangseinheit 8 des Endoroboters 5 übermittelt die Kamerabilder an den Rechner 13, der mittels Bildverarbeitungsalgorithmen bereits bestrahlte Bereiche 4 der Läsion erkennt und über das Magnetfeldsteuerungssystem durch Schwenken bzw. Bewegen des Endoroboters 5 entlang der X- und/oder Y-Achse den Laserstrahl auf noch nicht bestrahlte Läsionsbereiche sowie auf einen saumförmigen Randbereich richtet.
Die Kombination beider Regelkreise (Regelkreis 1 - Fig. 3a und Regelkreis 2 - Fig. 3b) ermöglicht einen automatisierten und optimierten läsionspezifischen Bestrahlungsverlauf durch den in Verbindung mit einer ebenso rechnerbasierten automatisierten Läsionserkennung eine Vielzahl von Läsionen in kürzester Zeit koaguliert werden können.
In unterschiedlichen (vorteilhaften) Ausführungsformen der Erfindung soll es unter anderem dem Benutzer auch möglich sein, in das automatisierte rechnerbasierte Geschehen auf unterschiedliche Weise zu unterschiedlichen Zeitpunkten einzugreifen. Folgende Ausführungsformen sind denkbar:
   1. Der Benutzer steuert den Endoroboter von Läsion zu Läsion und initialisiert jeweils den Bestrahlungsverlauf.
   2. Der Benutzer gibt möglicherweise über eine Menüauswahl auf dem Rechner den Verlauf der Flächenleistungsdichte während des Bestrahlungsverlaufes vor.
   3. Der Benutzer entscheidet aufgrund einer Merkmalsveränderung (z. B. Farbveränderung) der bestrahlten Läsion, ob die Bestrahlung bereits eine hinreichende Wirkung erzielt hat oder ob eine Weiterführung mit den bisherigen oder veränderten Bestrahlungsparametern (Laserbestrahlungsfläche, Frequenz, Bestrahlungsdauer) geboten ist.
   4. Dem jeweiligen automatisierten Bestrahlungslauf geht eine jeweilige, auf dem Bildschirm des Rechners vom Benutzer durchgeführte Segmentierung der zu bestrahlenden Läsion voraus.
Generell ist es insbesondere auch aus Sicherheitsgründen vorteilhaft, die Automatisierung der Läsionerkennung und Bestrahlung so zu gestalten, dass dem Benutzer eine Planung bzw. ein Eingreifen in sämtliche Phasen des rechnerbasierten Geschehens möglich ist.
Zusammengefasst kann gesagt werden, das bei der teilweise sehr großen Anzahl von Läsionen im Gastrointestinaltrakt eine Automatisierung der Läsionerkennung sowie der Läsionbestrahlung (Koagulation) einen von den Ärzten lange herbeigesehnten Wunsch erfüllt.
Das erfindungsgemäße Verfahren und System findet, wie bereits mehrfach erwähnt, seine hauptsächliche Anwendung im Bereich des Gastrointestinaltraktes, insbesondere des Dünndarmes. Dort tritt allerdings das häufige Problem auf, dass der Endoroboter durch die Darmwand so weit fixiert ist, dass er durch äußere Magnetfelder (Magnetfeldsteuersystem) nicht mehr oder nur sehr schwer gedreht werden kann. Insbesondere der Regelkreis 2 ist dadurch erheblich eingeschränkt.
Ein weiterer Aspekt der vorliegenden Erfindung besteht daher darin, den ablationsfähigen Laser im Endoroboter so auszubilden bzw. so anzuordnen, dass der Laserstrahl nicht ausschließlich durch Bewegung des Endoroboters justiert werden kann, sondern relativ zum Endoroboter geschwenkt, verschoben oder sogar an unterschiedlichen Positionen erzeugt werden kann. Dabei soll die gleiche Regelschleife aus Bilderkennung und Steuersignalen, wie oben beschrieben (Fig. 3a und 3b), verwendet werden.
Erfindungsgemäß soll der therapeutische Laser daher relativ zum Endoroboter entweder so beweglich angebracht sein, dass der Laserstrahl auf das Zielgewebe ausgerichtet werden kann ohne den Endoroboter selbst bewegen oder schwenken zu müssen, oder aber es wird alternativ zur Ausrichtung des Lasers eine Blende oder Linse im Inneren des Endoroboters so gesteuert bewegt, dass eine entsprechende Bewegung des Laserstrahles relativ zum Endoroboter erfolgt. Eine weitere dritte Möglichkeit wäre die Anordnung eines dreidimensionalen Laser-(Dioden)-Arrays im Inneren des Endoroboters, wobei der Laserstrahl ausschließlich durch Aktivierung derjenigen Laser erzeugt wird, durch die das Zielgewebe am besten getroffen wird.
Eine Beweglichkeit und räumliche Ausrichtung nach einer der ersten beiden erfindungsgemäßen Möglichkeiten kann durch alle bekannten miniaturisierbaren elektromechanischen Mechanismen erfolgen. Gedacht ist hierbei insbesondere an Motoren, bewegliche Membrane, Formgedächtnislegierungen, elektrisch kontraktierbare Polymere, Piezoaktoren usw. Auch das bereits vorhandene Magnetfeldsteuerungssystem kann verwendet werden, um den Laser relativ zum Endoroboter auszurichten, in dem der Laser beweglich aufgehängt und starr mit einem (Stab-) Magneten bzw. magnetisierbarem Material - der bzw. das nicht mit dem Stabmagneten des Endoroboter-Steuergerätes verwechselt werden darf - gekoppelt wird. Das äußere Magnetfeld kann so eingestellt werden, dass der Laser mittels des gekoppelten Magneten in eine vorgegebene Richtung gesteuert wird. Hierbei ist entweder der Endoroboter selbst, z. B. durch die Darmwand, so fixiert, dass er durch das steuernde Magnetfeld keine Lageveränderung erfährt oder die Masse bzw. Trägheit des gekoppelten Lasermagnetsystems ist so viel geringer als die des Endoroboters, dass zur Steuerung des Lasers ein erheblich schwächeres Magnetfeld als zur Steuerung des Endoroboters notwendig ist, ausreicht, um den Laser zu justieren.
Es sei erwähnt, dass die beschriebenen Steuermechanismen nicht auf den Laser des Endoroboters beschränkt sind. Vielmehr können auch andere Komponenten des Endoroboters (z. B. die Videokamera, andere Sensoren oder therapeutische Mittel) relativ zum Endorobotergehäuse mittels der beschriebenen Mechanismen bewegt und ausgerichtet werden.
Weiterhin ist anzumerken, dass Regelkreis 1 und Regelkreis 2 nicht notwendigerweise mit der Übersendung von Signalen aus dem Körperinneren an einen Rechner außerhalb des Endoroboters verknüpft sein muss. Die gesamte Bildverarbeitung und Steuerung des Endoroboters bzw. des Laserstrahles könnte auch durch einen im Endoroboter integrierten Prozessor vorgenommen werden. Auch die Kombination eines Rechners außerhalb des Patienten mit einem im Endoroboter integrierten Rechner ist denkbar.

## Patentansprüche

1. Verfahren zur automatisierten Lokalisierung von Läsionen
(1) im Gastrointestinaltrakt zur Therapie mit Laserlicht eines Endoroboters (5), aufweisend die folgenden Schritte:
- Aufnehmen von Oberflächenbildern des Gastrointestinaltraktes mit der Kamera (7) des Endoroboters (5)
- Übermitteln der Oberflächenbilder an einen Rechner (13)
- Analysieren der Oberflächenbilder durch den Rechner (13) nach zu bestrahlenden Läsionen auf Basis läsionspezifischer Merkmale
- Berechnen von Regelsignalen durch den Rechner (13) auf Basis einer erkannten Läsion,
- Übermitteln der Regelsignale an ein Endoroboter-Steuergerät und/oder an ein im Endoroboter befindliches Lasersystem (10), und
- Einstellen der berechneten Laserstrahl-Ausrichtung und Laserbestrahlungsflächengröße durch das Endoroboter-Steuergerät und/oder durch das Lasersystem (10) auf Basis der Regelsignale.

2. Verfahren nach Anspruch 1,
**dadurch gekennzeichnet,**
**dass** ein geregeltes Bestrahlen der erkannten Läsion erfolgt, aufweisend die folgenden Schritte:
- Bestrahlen der vom Rechner (13) auf Basis läsionspezifischer Merkmale erkannten Läsion mit dem Laser (10) des Endoroboters (5)
- Messen aktueller läsionspezifischer Merkmale der bestrahlten Fläche durch die Kamera (7) nach Abschalten des Lasers (10)
- Ermitteln der Größe der Laserbestrahlungsfläche (2) durch den Rechner (13) auf Basis der gemessenen aktuellen läsionspezifischen Merkmale
- Einstellen der ermittelten Laserbestrahlungsflächengröße durch Positionieren des Endoroboters (5) orthogonal zur Läsionoberfläche
- Kontinuierliches oder pulsartiges Bestrahlen der Läsionoberfläche, Messen und Überwachen der Größe sowie der aktuellen läsionspezifischen Merkmale der Laserbestrahlungsfläche (2) auf der Läsionoberfläche.

3. Verfahren nach einem der Ansprüche 1 bis 2,
**dadurch gekennzeichnet,**
**dass** die Laserbestrahlungsflächengröße in Abhängigkeit der gemessenen aktuellen läsionspezifischen Merkmale der Laserbestrahlungsfläche (2) auf der Läsionoberfläche durch Einstellen der Entfernung des Endoroboters (5) zur Läsionoberfläche variiert wird.

4. Verfahren nach Anspruch 1,
**dadurch gekennzeichnet,**
**dass** die Laserbestrahlungsflächengröße in Abhängigkeit der gemessenen aktuellen läsionspezifischen Merkmale der Laserbestrahlungsfläche (2) auf der Läsionoberfläche durch Variation des Lasersystems variiert wird.

5. Verfahren nach einem der Ansprüche 1 bis 4,
**dadurch gekennzeichnet,**
**dass** die läsionspezifischen Merkmale die Farbe, die Form sowie die Textur einer spezifischen Läsion (1) betreffen.

6. Verfahren nach einem der vorhergehenden Ansprüche,
**dadurch gekennzeichnet,**
**dass** die läsionspezifischen Merkmale mit Läsionsmerkmalen in einer dem Rechner (13) zugänglichen Bibliothek bzw. in einer Bibliothek des Rechners (13) verglichen werden.

7. Verfahren nach einem der vorhergehenden Ansprüche,
**dadurch gekennzeichnet,**
**dass** die Bestrahlung mit Laserlicht zur Messung der Laserbestrahlungsflächengröße sowie zur Messung der läsionspezifischen Merkmale jederzeit bzw. in periodischen Intervallen unterbrochen bzw. gestoppt wird.

8. Verfahren nach einem der vorhergehenden Ansprüche,
**dadurch gekennzeichnet,**
**dass** das Aufnehmen von Oberflächenbildern, das Übermitteln an den Rechner (13) sowie das Analysieren durch den Rechner (13) kontinuierlich erfolgt.

9. Verfahren nach einem der vorhergehenden Ansprüche,
**dadurch gekennzeichnet,**
**dass** durch transversales Verschieben des Laserstrahles in der XY-Ebene bzw. durch Schwenken des Laserstrahles relativ zur Läsion-Endoroboter-Verbindungslinie (Z-Achse) durch entsprechende Bewegung des Endoroboters (5) in Kombination mit dem kontrollierten Positionieren in Z-Richtung des Endoroboters (5) die gesamte Läsionoberfläche mit definierter Flächenleistungsdichte des Lasers (10) bestrahlt wird.

10. Verfahren nach Anspruch 9,
**dadurch gekennzeichnet,**
**dass** die Flächenleistungsdichte in Abhängigkeit von der Art der Läsion (1) über die Zeit vom Rechner (13) vorgegeben wird.

11. Verfahren nach einem der Ansprüche 8 bis 10,
**dadurch gekennzeichnet,**
**dass** das transversale Verschieben des Laserstrahles in der XY-Ebene bzw. das Schwenken des Laserstrahles relativ zur Z-Achse durch räumliche Ausrichtung des Lasers (10) relativ zum Endoroboter (5) erfolgt.

12. Verfahren nach Anspruch 11,
**dadurch gekennzeichnet,**
**dass** die räumliche Ausrichtung des Lasers (10) relativ zum Endoroboter (5) durch miniaturisierte elektromechanische Mechanismen, wie bewegliche Membrane, Formgedächtnislegierungen, elektrisch kontraktierbare Polymere, Piezoaktoren usw. erfolgt.

13. Verfahren nach Anspruch 11,
**dadurch gekennzeichnet,**
**dass** die Laserstrahlbewegung durch Verschiebung einer Blende bewirkt wird.

14. Verfahren nach Anspruch 11,
**dadurch gekennzeichnet,**
**dass** die Laserstrahlbewegung durch Aktivieren ausgewählter Laserdioden eines im Endoroboter (5) integrierten 3D-Laser-Dioden-Arrays bewirkt wird.

15. Verfahren nach Anspruch 11,
**dadurch gekennzeichnet,**
**dass** die Laserstrahlbewegung durch ein Magnetfeldsteuerungssystem bewirkt wird, in dem der im Endoroboter (5) integrierte Laser (10) an einen beweglichen Miniaturmagneten bzw. an magnetisierbarem Material gekoppelt ist, welcher bzw. welches nicht zur Navigation des Endoroboters dient, und welcher bzw. welches durch Anlegen eines äußeren Magnetfeldes geschwenkt bzw. verschoben werden kann.

16. Verfahren nach einem der Ansprüche 11 bis 15,
**dadurch gekennzeichnet,**
**dass** eine Ausrichtung weiterer Komponenten des Endoroboters (5), z. B. die Kamera (7), mittels der Steuerprinzipien gemäß der Ansprüche 10 bis 13 erfolgt.

17. System zur Durchführung eines Verfahrens nach einem der Ansprüche 1 bis 16.

18. System nach Anspruch 17,
**dadurch gekennzeichnet,**
**dass** der Rechner außerhalb des zu untersuchenden Patienten platziert ist.

19. System nach Anspruch 17,
**dadurch gekennzeichnet,**
**dass** der Rechner im Inneren des Endoroboters integriert ist.
